# EUROPEAN PATENT APPLICATION

(11) **EP 4 445 946 A1**
(43) Date of publication of application: **16.10.2024**
(21) Application number: 23167329.4
(22) Date of filing: 11.04.2023
(51) Int. Cl.: A61N 1/372, A61B 5/00, A61N 1/36, A61N 1/37

(54) **SYSTEM AND METHOD FOR IDENTIFYING AN INFECTION INDICATION IN A PATIENT**

(71) Applicant: BIOTRONIK SE & Co. KG, 12359 Berlin (DE)
(72) Inventor: Wang, Xinlong, Arlington, 76013 (US); Reddy, Ravi Kiran Kondama, Portland, 97221 (US); Whittington, R. Hollis, Portland, 97202 (US)
(74) Representative: Biotronik Corporate Services SE

(57) **Abstract**

A system for performing a therapeutic and/or diagnostic function in a patient (P) comprises an implantable medical device (1) comprising an optical sensing arrangement (15) comprising at least one light-detecting device (151, 151-1... 151-4) for sensing a light detection signal. A processing arrangement is configured to process an output value of said at least one light-detecting device (151, 151-1... 151-4) indicative of said light detection signal for identifying an infection indication.

## Description

The instant invention generally relates a system for performing a therapeutic and/or diagnostic function in a patient and to a method for identifying an infection indication in a patient.

A system of the type concerned herein comprises an implantable medical device configured for implantation in a patient and a processing arrangement configured to process signals. The processing arrangement may, full or in part, be part of the implantable medical device, or may be outside of the implantable medical device, for example in an external device in communication connection with the implantable medical device.

An implantable medical device of the type concerned herein generally may comprise an arrangement of electrode poles configured to at least one of the output electrical stimulation signals for effecting a stimulation action or sense electrical excitation signals.

The implantable medical device may for example be a cardiac pacemaker, an implantable cardioverter defibrillator, a sensor device such as a bio-sensor, or a monitoring device. The implantable medical device may serve a cardiac diagnostic and/or therapeutic function. In other embodiments the implantable medical device may serve a neuro-stimulation function or the like.

For example, the implantable medical device may be a cardiac monitoring device which is configured to record electrocardiogram signals and to communicate recorded electrocardiogram signals or information derived from recorded electrocardiogram signals to an external device in the context of a home monitoring system.

An implantable medical device as for example described in EP 3 278 836 B1 may for example comprise a housing and an arrangement of electrode poles arranged on the housing. The electrode poles herein are arranged on the housing of the implantable medical device such that the electrode poles are aligned along a longitudinal axis along which the implantable medical device extends. The electrode poles may for example be formed by housing segments which are made from an electrically conductive material such as a metal material and are exposed to the outside such that they may be brought into electrical contact with surrounding tissue in order to establish an electrical coupling to the tissue in an implanted state of the implantable medical device.

Implanting an implantable medical device in a patient comes with the inherent risk of infection at the site of implantation. An incidence rate for the occurrence of an infection at an implantation site of an implantable medical device in the past has been found to be in the range of 1 to 2%, the incidence rate possibly increasing if a replacement of the implantable medical device, for example, due to a malfunction of the implantable medical device, becomes necessary.

If an infection at an implantation site of an implantable medical device occurs in a patient, this may have severe consequences for the patient. It has been found that upon the occurrence of an infection at an implantation site of an implantable medical device, a 30-day mortality rate may be in the order of 5%, a 1-year mortality rate may be in the order of 25%, and a 3-year mortality rate may be in the order of 50%. If an infection at an implantation site of the implantable medical device is found, it typically becomes necessary to remove the implantable medical device, the removal procedure bearing a significant risk of potentially life-threatening complications for the patient.

For these reasons it is of great importance that an infection at an implantation site of an implantable medical device is diagnosed as timely as possible in order to be able to initiate treatment of the infection as fast as possible. Current approaches for identifying an infection herein rely e.g. on a physical examination of the patient, which however typically takes place only after the occurrence of specific symptoms indicative of an infection. Further, periodic blood culture testing may be performed, the blood culture testing however being invasive and cumbersome. Other approaches may involve CT or PET examinations, which generally expose the patient to radiation. Echocardiography may be used to identify an infection, but is inaccurate and requires experienced interpretations. All of the above approaches, in addition, come with the drawback that they can be performed only in clinical environments requiring a patient to visit a clinic.

It is an object of the instant invention to provide a system for performing a therapeutic and/or diagnostic function in a patient and a method for identifying an infection indication which allows in an easy and reliable manner to timely identify an infection indication indicative of infection at an implantation site of an implantable medical device in a patient.

In one aspect, a system for performing a therapeutic and/or diagnostic function in a patient comprises an implantable medical device comprising an optical sensing arrangement comprising at least one light-detecting device for sensing a light detection signal. The system further comprises a processing arrangement configured to process an output value of the at least one light-detecting device indicative of the light detection signal for identifying an infection indication.

Accordingly, the implantable medical device comprises an optical sensing arrangement comprising at least one light-detecting device for sensing a light detection signal. The optical sensing arrangement is configured to sense the light detection signal in a region outside of the implantable medical device, the light detection signal hence stemming from a light signal in the vicinity of the implantable medical device. The processing arrangement is configured to process output values of at least one light-detecting device of the optical sensing arrangement in order to identify an infection indication indicative of an infection at the implantation site of the implantable medical device.

In one embodiment, the implantable medical device comprises processing circuitry implementing at least part of the processing arrangement. In particular, in one embodiment the processing of output values of the at least one light-detecting device of the optical sensing arrangement may be carried out entirely in the implantable medical device for identifying an infection indication indicative of an infection at the implantation site of the implantable medical device. In other embodiments, the processing circuitry of the implantable medical device carries out only some of the processing steps, wherein the implantable medical device may be in communication connection with an external device configured to rest outside of the patient for forwarding information relating to output values of the at least one light-detecting device or intermediate processing results to the external device for further processing to identify an infection indication.

In one embodiment, the output value of the at least one light-detecting device is indicative of a light intensity of the light detection signal detected by the at least one light-detecting device. The at least one light-detecting device hence senses light and outputs an output value indicative of a light intensity of a sensed light signal, wherein the output value is provided to the processing arrangement and is processed by the processing arrangement for identifying an infection indication indicative of an infection at the implantation site of the patient.

Within a measurement for identifying an infection indication, generally, a light emission signal is emitted by a light source, and the light detection signal is sensed by the at least one light-detecting device of the optical sensing arrangement in response to the emission of the light emission signal. Generally, the light emission signal is absorbed and scattered by tissue and exudate fluids in the vicinity of the implantable medical device, in particular within an implantation pocket in which the implantable medical device rests within the patient. The light detection signal as sensed by the at least one light-detecting device hence depends on absorption and scattering properties in the vicinity of the implantable medical device. As absorption and scattering properties may change with changing conditions at the implantation site of the implantable medical device, an infection indication may be determined based on sensed light detection signals. In particular, the processing arrangement may conclude for an infection indication and hence may identify said infection indication in case sensed light detection signals indicate conditions at the implantation site of the implantable medical device which are indicative of an infection at the implantation site.

The infection indication may in particular be a Boolean marker, e.g., having values of true or false, the infection indication being identified as true, based on sensed light detection signals, if it is found that an infection likely is present at the implantation site of the implantable medical device. In other embodiments, the infection indication may be a numeric value, for example relating to an intensity of a received light detection signal at a particular wavelength or in a wavelength range indicative of a deviation from a normal, non-infected state.

A light emission signal may be produced by different means.

In one embodiment, an external light source outside of the patient may be used for emitting a light emission signal into the patient, wherein the at least one light-detecting device of the optical sensing arrangement is configured to sense a light detection signal in response to the emission of the light emission signal by the external light source.

In another embodiment, the optical sensing arrangement comprises at least one light-emitting device for emitting a light emission signal. The optical sensing arrangement hence is configured for generating and outputting a light emission signal, in response to which the at least one light-detecting device detects a light detection signal relating to the light emission signal for outputting an output value to be processed by the processing arrangement. Light emission and light reception hence take place at the implantable medical device, the at least one light emitting device being configured to emit a light emission signal into a region outside of the implantable medical device and the at least one light detecting device being configured to receive a light detection signal from said region outside of the implantable medical device.

The at least one light-emitting device may for example be a light-emitting diode (LED).

The at least one light-detecting device may for example be a photodetector, such as a photodiode.

One embodiment, the optical sensing arrangement comprises a multiplicity of light emitting devices. A first of the multiplicity of light emitting devices is configured to emit a first light emission signal at a first wavelength or in a first wavelength range. A second of the multiplicity of light emitting devices is configured to emit a second light emission signal at a second wavelength or in a second wavelength range different than the first wavelength or the first wavelength range. A third of the multiplicity of light emitting devices may be configured to emit a third light emission signal different in wavelength than the first and the second light emission signal, wherein further light emitting devices for emitting further, different light emission signals may be present. For example, the optical sensing arrangement may comprise a number in between 2 to 12, for example, 4 or 8 light emitting devices configured to emit light emission signals at different wavelengths or in different wavelength ranges.

By providing different light emitting devices, each light emitting device being configured to emit a light emission signal at a particular wavelength or in a particular wavelength range, a spectroscopic analysis of light absorption and scattering properties in the vicinity of the implantable medical device is enabled. In particular, in response to the emission of the first light emission signal by the first light emitting device the at least one light-detecting device detects a first light detection signal indicative of a light reception in response to the first light emission signal at the first wavelength or in the first wavelength range. In response to the emission of the second light emission signal by the second light emitting device the at least one light-detecting device detects a second light detection signal indicative of a light reception in response to the second light emission signal at the second wavelength or in the second wavelength range. In response to further light emission signals emitted by further light emitting devices, further light detection signals may be obtained. Output values of the at least one light-detecting device hence are indicative of received light intensities at different wavelengths or in different wavelength ranges, such that spectroscopic information of light absorption and light scattering properties at different wavelengths or in different wavelength ranges is obtained.

In one embodiment, the multiplicity of light-emitting devices may be grouped around a single light-detecting device. The light-detecting device, for example a photodetector, may be configured for light reception in a broad wavelength range covering the particular wavelengths or wavelength ranges of the multiplicity of light emitting devices. In particular, the light-emitting devices and the light-detecting device may be arranged at a surface of a housing of the implantable medical device, the light-emitting devices being grouped around the light-detecting device along a plane of the housing of the implantable medical device. In particular, the light-detecting device may be arranged in the center, and the multiple light-emitting devices may be arranged around the central light-detecting device.

In one embodiment, the optical sensing arrangement comprises a multiplicity of light-detecting devices. A first of the multiplicity of light-detecting devices is configured to sense a first light detection signal at a first wavelength or in a first wavelength range. A second of the multiplicity of light-detecting devices is configured to sense a second light detection signal at a second wavelength or in a second wavelength range different than the first wavelength or the first wavelength range. A third light-detecting device may be configured to sense a third light detection signal at a third wavelength or in a third wavelength range, wherein further light-detecting devices for detecting light detection signals at further wavelengths or in further wavelength ranges different than the other wavelengths or wavelength ranges may be present.

Rather than emitting spectroscopically different light emission signals, different light detection signals at different wavelengths or in different wavelength ranges may be received by a multiplicity of light-detecting devices, such that spectroscopic information relating to the absorption and scattering properties in the vicinity of the implantable medical device is obtained by the different light detection signals associated with different wavelengths or different wavelength ranges.

The multiplicity of light-detecting devices may, in one embodiment, be grouped around a single light-emitting device. The light emitting device, for example an LED, may be configured for light emission in a broad wavelength range covering the particular wavelengths or wavelength ranges of the multiplicity of light-detecting devices. In particular, the light-emitting device and the multiple light-detecting devices may be arranged at a surface of a housing of the implantable medical device, the light-detecting device being grouped around the light-emitting device along a plane of the housing of the implantable medical device. In particular, the light-emitting device may be arranged in the center, and the multiple light-detecting devices may be arranged around the central light-emitting device.

In one embodiment, the processing arrangement is configured to process output values of the at least one light-detecting device associated with different wavelengths or different wavelength ranges for identifying the infection indication in an infection indication measurement. The output values may be obtained using a single light-detecting device by detecting light detection signals in response to different light emission signals emitted by different light-emitting devices. In another embodiment, the output values may be obtained using multiple different light-detecting devices by detecting light detection signals in response to a broadband light emission signal emitted by a single light-emitting device. By processing output values associated with light detection signals at different wavelengths or in different wavelength ranges, optical absorption and scattering properties in the vicinity of the implantable medical device may be assessed. By identifying optical absorption and scattering properties indicative of an infection situation, the infection indication may be identified.

A first output value may for example be associated with a light detection signal at a wavelength of 550 nm or in a wavelength range around 550 nm. A second output value may for example be associated with a light detection signal at a wavelength of 600 nm or in a wavelength range around 600 nm. A third output value may for example be associated with a light detection signal at a wavelength of 650 nm or in a wavelength range around 650 nm. A fourth output value may for example be associated with a light detection signal at a wavelength of 900 nm or in a wavelength range around 900 nm.

An infection at an implantation site, in particular within an implantation pocket in which the implantable medical device is implanted in a patient, will cause a characteristic change of properties at the implantation site, which also comes with a change of optical transmission and reflection properties, caused by a change in absorption and scattering conditions. In particular, in a healthy, non-infected state generally a clear serous fluid is found at an implantation site which does not significantly change the transmission and reflection properties. In an infected state, in contrast, different transmission and reflection properties in comparison to a healthy state can be found, caused by an opaque or translucent exudate fluid at the implantation site, for example a purulent fluid indicative of an infection at the infection site. Based on a change in the output values it hence may be concluded for an infection at the implantation site of the implantable medical device, such that an infection indication may be determined and an infection alert may be triggered.

In one embodiment, the processing arrangement may be configured to control the optical sensing arrangement to sequentially obtain, in an infection indication measurement, output values associated with different wavelengths or different wavelength ranges for identifying the infection indication. Output values associated with light detection signals at different wavelengths or in different wavelength ranges hence are obtained sequentially, by sequentially controlling a multiplicity of light emitting devices to emit different light emission signals, or by sequentially controlling a multiplicity of light detection devices to sequentially detect different light detection signals. Within an infection indication measurement for measuring an infection indication, hence, a multiplicity of output values is sequentially measured, wherein the output values are processed in order to identify a deviation from a healthy state potentially indicative of an infection at the implantation site.

In one embodiment, the processing arrangement is configured to associate the output values associated with different wavelengths or different wavelength ranges with a set of reference values for identifying the infection indication. The set of reference values are in particular associated with a healthy, non-infected state, such that based on a deviation from the reference values it may be identified that an infection is present.

The set of reference values may for example be used as a reference, wherein the output values associated with light detection signals at different wavelengths or in different wavelength ranges may be compared to the set of reference values. If it is found for example that at least one output value deviates from an associated reference value by e.g., more than a certain margin, it is concluded that an infection is present and an infection indication is correspondingly identified.

In one embodiment, the set of reference values may be used to normalize the output values in an infection indication measurement. If it is found that at least one output value deviates from a value of 1, for example is increased above a value of 1, by more than a certain margin it may be concluded that an infection is present, and an infection indication is correspondingly identified.

Infection indication measurements may be repeated regularly, for example once per day, once per week, or once per month, such that monitoring for a possible infection takes place regularly.

Based on regular infection indication measurements, also, a change in optical properties may be observed, wherein it may be concluded for an infection if it is found, based on the output values in an infection indication measurement, that a deviation from reference values indicative of a normal, non-infected, healthy a state has become significant as a result of a corresponding trend.

In one embodiment, the processing arrangement is configured, in a calibration measurement, to control the optical sensing arrangement for obtaining output values associated with different wavelengths or different wavelength ranges to record the reference values to be used in infection indication measurements subsequent to conducting the calibration measurement. For example, a calibration measurement may be performed upon the initial implantation of the implantable medical device at the implantation site. Within the calibration measurement, reference values are determined as indicative of a normal, healthy state. The calibration measurement may be repeated regularly, for example once per month or once every few months.

In one embodiment, the optical sensing arrangement comprises a temperature sensor for sensing a temperature measurement value indicative of a temperature in the vicinity of the optical sensing arrangement.

The temperature sensor may in particular be used to identify if, due to the optical measurements for determining an infection indication, it comes to a local heating at the implantation site of the implantable medical device. If it is found that, during an infection indication measurement, the temperature rises at the implantation site above a certain value, the measurement may be terminated in order to avoid the effects of local heating.

Temperature measurement values as obtained using the temperature sensor may also be associated with output values as obtained using the optical sensing arrangement. For example, if a rise in temperature is observed, without a change in optical properties as indicated by the output values, this may indicate the presence of a systemic infection of the patient, rather than a local infection at the implantation site of the implantable medical device. If, however, a temperature rise is observed by the temperature sensor at the implantation site with a corresponding change in optical properties as identified based on output values of the optical sensing arrangement, this may additionally confirm the presence of a local infection at the infection site. Temperature readings of the temperature sensor hence may be used, in association with output values of the optical sensing arrangement, to distinguish between a local infection at the implantation site and a systemic infection of the patient.

The temperature sensor may for example comprise a thermistor. The temperature sensor is arranged in close vicinity to the at least one light-detecting device of the optical sensing arrangement.

In one embodiment, the implantable medical device comprises communication circuitry for establishing a communication with an external device for communicating at least one of information relating to output values of the optical sensing arrangement or information relating to processing results for identifying the infection indication to the external device. Based on the amount of processing that is done immediately in the implantable medical device, the information communicated from the implantable medical device to the external device may vary. If the entire processing for identifying the infection indication is carried out by the implantable medical device and its processing circuitry, information relating to the infection indication may be communicated to the external device, for example within the context of a home monitoring system. If only some of the processing of the output values is carried out by the processing circuitry of the implantable medical device, wherein further processing is carried out by the external device or another device for identifying the infection indication, information relating to intermediate processing results of the processing of the output values may be communicated from the implantable medical device to the external device for further processing at the external device or at another device, for example in a cloud.

In another aspect, a method for identifying an infection indication in a patient comprises: sensing, using at least one light-detecting device of an optical sensing arrangement of an implantable medical device, a light detection signal; and processing an output value of the at least one light-detecting device indicative of the light detection signal for identifying an infection indication.

The advantages and advantageous embodiments as described above for the system equally apply also to the method, such that it shall be referred to the above.

Within the method, some or all of the processing may be carried out by the implantable medical device. It is conceivable that the implantable medical device is only used to obtain output values of the optical sensing arrangement, wherein the actual processing of the output values for identifying the infection indication is carried out by processing equipment outside of the implantable medical device, for example in an external device in the context of a home monitoring system or on a cloud server device. In another embodiment, all of the processing is carried out by the implantable medical device, such that the implantable medical device determines the infection indication and communicates information with respect to the infection indication to an external device, for example within the context of a home monitoring system.

The various features and advantages of the present invention may be more readily understood with reference to the following detailed description and the embodiments shown in the drawings. Herein,
- Fig. 1: shows a schematic drawing of an implantable medical device implanted in a patient;
- Fig. 2: shows a schematic drawing of an implantable medical device;
- Fig. 3: shows a schematic drawing of an optical sensing arrangement of the implantable medical device;
- Fig. 4A: shows a schematic drawing of an operation of the optical sensing arrangement, during light emission of a first light-emitting device and light reception by a light-detecting device;
- Fig. 4B: shows a schematic drawing of an operation of the optical sensing arrangement, during light emission of a second light-emitting device and light reception by the light-detecting device;
- Fig. 5: shows a schematic flow diagram of an operation for conducting an infection indication measurement for identifying an infection indication;
- Fig. 6A: shows a curve of light intensities as a function of wavelength, corresponding to a set of reference values as obtained during a calibration measurement;
- Fig. 6B: shows different curves of light intensities as a function of wavelength, obtained in different infection indication measurements;
- Fig. 7A: shows temperature measurement values of a temperature sensor of the optical sensing arrangement as obtained for different infection indication measurements;
- Fig. 7B: shows a curve of an example of temperature measurement values of a temperature sensor of the optical sensing arrangement as obtained during an infection indication measurement; and
- Fig. 8: shows a schematic drawing of another embodiment of an optical sensing arrangement.

Subsequently, embodiments of the invention shall be described in detail with reference to the drawings. In the drawings, like reference numerals designate like structural elements.

It is to be noted that the embodiments are not limiting or limited for the invention, but merely represent illustrative examples.

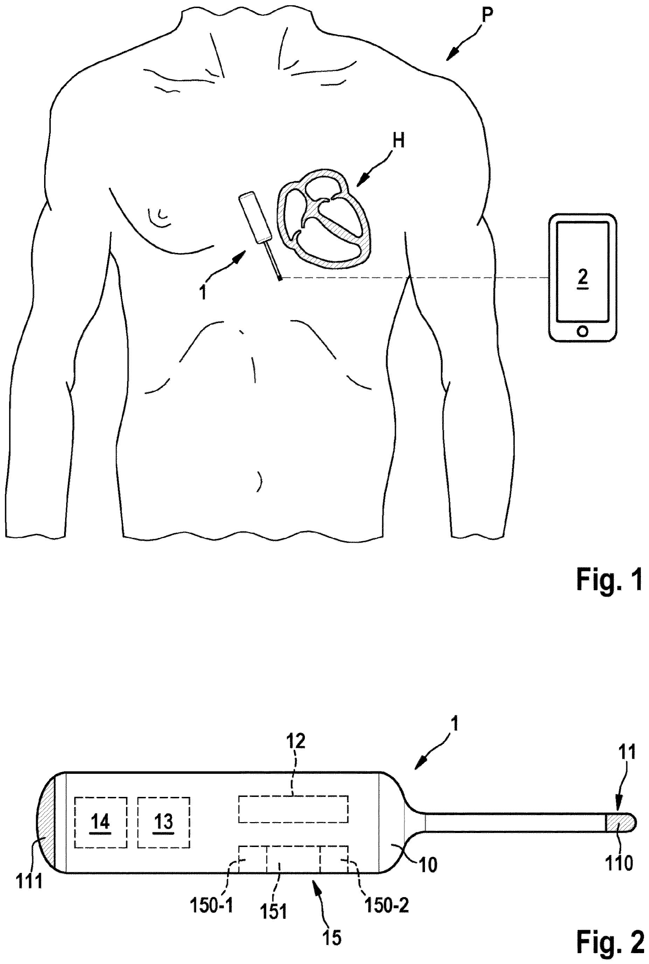

Referring to Fig. 1, in one embodiment a system comprises an implantable medical device 1 implanted (for example subcutaneously) into a patient for serving a therapeutic and/or diagnostic function. The implantable medical device 1 may for example be implanted subcutaneously into a patient P for monitoring the cardiac activity of the patient's heart H. The implantable medical device 1, for this, comprises an arrangement of electrode poles which are used to couple to surrounding tissue and to sense electrocardiogram signals originating from the heart H.

The system furthermore comprises an external device 2 external to the patient P and being in communication connection with the implantable medical device 1.

The implantable medical device may for example be a monitoring device for sensing electrocardiogram signals. In another embodiment, the implantable medical device 1 may be a pacemaker device, a cardioverter defibrillator device, or a sensor device such as a biosensor. In yet another embodiment the implantable medical device may be a stimulation device such as a neuro-stimulation device.

Referring now to Fig. 2, in one embodiment the implantable medical device 1 comprises a housing 10 and an electrode arrangement 11. The electrode arrangement 11 comprises a multiplicity of electrode poles 110, 111 arranged at different locations on the housing 10 or on one or multiple leads extending from the housing 10 towards a region of interest, such as into the patient's heart. By means of the electrode arrangement 11 the implantable medical device 1 may for example be configured to sense electrical signals and/or to output electrical stimulation signals, such as pacing signals or the like, for achieving a diagnostic and/or therapeutic function within the patient P.

The implantable medical device 1 comprises a processing circuitry 12 configured for controlling an operation of the implantable medical device 1 and for processing signals. In addition, the implantable medical device 1 comprises an energy storage 13, in particular an electrochemical battery. A communication circuitry 14 enables the implantable medical device 1 to establish a communication connection to an external device 2, for example within the context of a home monitoring system.

The implantable medical device 1 is configured for implantation at an implantation site in a patient. For implantation the implantable medical device 1 for example is inserted into a subcutaneous pocket in the patient P and, in an implanted state, rests within the pocket for operating within the patient P over an extended period of time, for example several months or even years.

Implantation of an implantable medical device 1 in a patient P comes with the inherent risk of an infection at the implantation site, an infection bearing a severe, possibly mortal risk for the patient. As the occurrence of an infection at the implantation site of an implantable medical device 1 comes with a substantial mortality risk, it is crucial that countermeasures such as a removal of the implantable medical device 1 from an implantation site and medicational treatment of the patient take place in a timely fashion upon development of the infection. An infection at an implantation site of the implantable medical device hence shall be detected in a timely fashion.

For this, the implantable medical device 1 in the embodiment of Fig. 2 comprises an optical sensing arrangement 15 configured to process optical signals indicative of optical properties in the vicinity of the implantable medical device 1 such that, based on the optical signals, an infection indication may be determined and may be used to trigger an alert in case an infection is detected.

The optical sensing arrangement 15 comprises, in the shown embodiment, a multiplicity of light emitting devices 150-1, 150-2, and a light-detecting device 151. By means of the light emitting devices 150-1, 150-2 light emission signals are emitted into a region outside of the implantable medical device 1, wherein in response to the emission the light-detecting device 151 senses light detection signals and outputs corresponding output signals for processing by the processing circuitry 12.

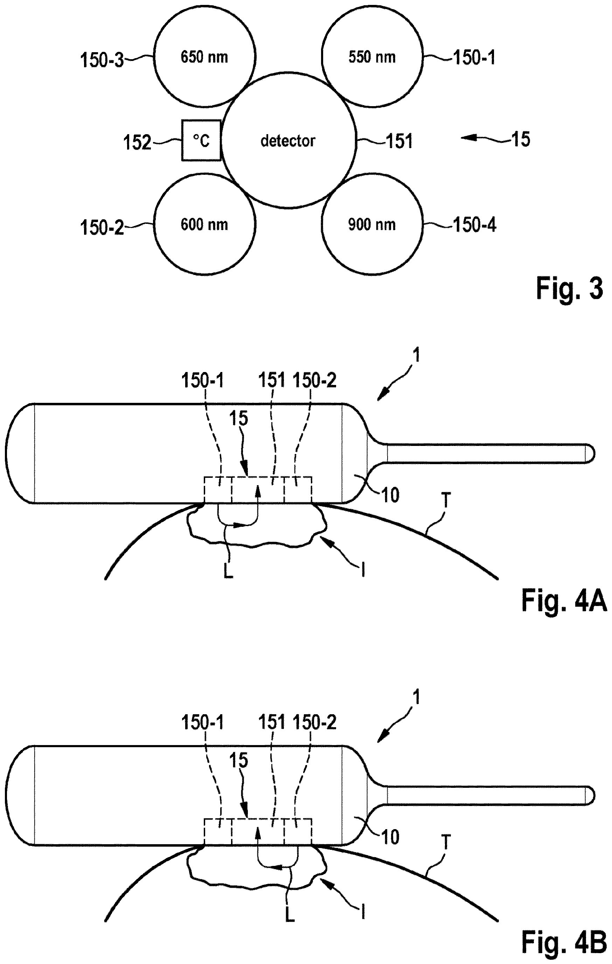

Referring now to Fig. 3, in one embodiment the optical sensing arrangement 15 comprises a multiplicity of light emitting devices 150-1 ... 150-4 and a single light-detecting device 151. The light-emitting devices 150-1... 150-4 are grouped around the light-detecting device 151 such that the light-detecting device 151 is arranged centrally within the group of light-emitting devices 150-1... 150-4.

The light-emitting devices 150-1... 150-4 and the light-detecting device 151 are arranged on the housing 10, as visible from Fig. 2, such that light is emitted by the light-emitting devices 150-1 ... 150-4 towards the outside of the implantable medical device 1, and light is sensed by the light-detecting device 151 from the outside of the implantable medical device 1.

By means of the different light-emitting devices 150-1... 150-4, light emission signals at different wavelengths or in different wavelength ranges are generated and output by the optical sensing arrangement 15. In the embodiment of Fig. 3, a first light-emitting device 150-1 may for example output a light emission signal at a wavelength of 550 nm or in a wavelength range around 550 nm. A second light emitting device 150-2 may output a light emission signal at a wavelength of 600 nm or in a wavelength range around 600 nm. A third light emitting device 150-3 may output a light emission signal at a wavelength of 650 nm or in a wavelength range around 650 nm. A fourth light emitting device 150-4 may output a light emission signal at a wavelength of 900 nm or in a wavelength range around 900 nm and hence in the near-infrared light region.

Whereas the light emitting devices 150-1... 150-4 are configured for outputting narrow-band light signals at particular wavelengths or in particular wavelength ranges, the light-detecting device 151 is configured for a broadband light reception, such that by means of the single light-detecting device 151 light detection signals in response to the light emission signals of the light emitting devices 150-1... 150-4 may be sensed.

The light-detecting device 151 may for example be a photodetector, such as a photodiode.

The light emitting devices 150-1... 150-4 may for example be light emitting diodes, in short LEDs.

In addition, the optical sensing arrangement 15 comprises a temperature sensor 152 which outputs temperature measurement values indicative of a temperature in the vicinity of the optical sensing arrangement 15, in particular indicative of a temperature outside of the implantable medical device 1 in the vicinity of the optical sensing arrangement 15.

Making use of the optical sensing arrangement 15, infection indication measurements may be conducted for identifying an infection indication indicative of an infection at the implantation site of the implantable medical device 1. Within an infection indication measurement, the processing circuitry 12 may control the light emitting devices 150-1... 150-4 to sequentially emit light emission signals, in response to which the light-detecting device 151 senses associated light detection signals and outputs output values indicative of light intensities associated with the different light detection signals sensed in response to the light emission signals at the different wavelengths or in the different wavelength ranges.

Referring now to Fig. 4A, in a measurement step, the light emitting device 150-1 may for example output a light emission signal L, which is transmitted into and reflected by tissue T (e.g. of an infected region I) and exudate fluids in the region of the implantable medical device 1. In particular, the light emission signal L is to some extent absorbed and to some extent scattered at the tissue T and at fluids at the implantation site of the implantable medical device 1, such that only a portion of the light emission signal L is reflected towards the light-detecting device 151 and correspondingly is sensed at the light-detecting device 151. The light-detecting device 151 outputs an output value indicative of a light intensity of the sensed light in response to the light emission signal L as generated by the light emitting device 150-1.

In another measurement step, shown in Fig. 4B, another light emitting device 150-2 is controlled to output a light emission signal L, which again is partially absorbed and partially scattered, such that in response to the light emission signal L a light detection signal is sensed at the light-detecting device 151, which correspondingly outputs an output value indicative of the intensity of the sensed light in response to the light emission signal L.

By controlling the light emitting devices 150-1... 150-4 to generate light emission signals at different wavelengths or in different wavelength ranges, spectroscopic information is obtained indicative of optical transmission and reflection properties in the vicinity of the implantable medical device 1. Based on such spectroscopic information, which is obtained according to the output values of the light-detecting device 151 in response to the different light emission signals at the different wavelengths or in the different wavelength ranges, an infection indication may be determined and output, the infection indication indicating e.g. the presence of an infection at the implantation site as a Boolean variable or the likelihood of an infection at the implantation site as a risk (probability) parameter.

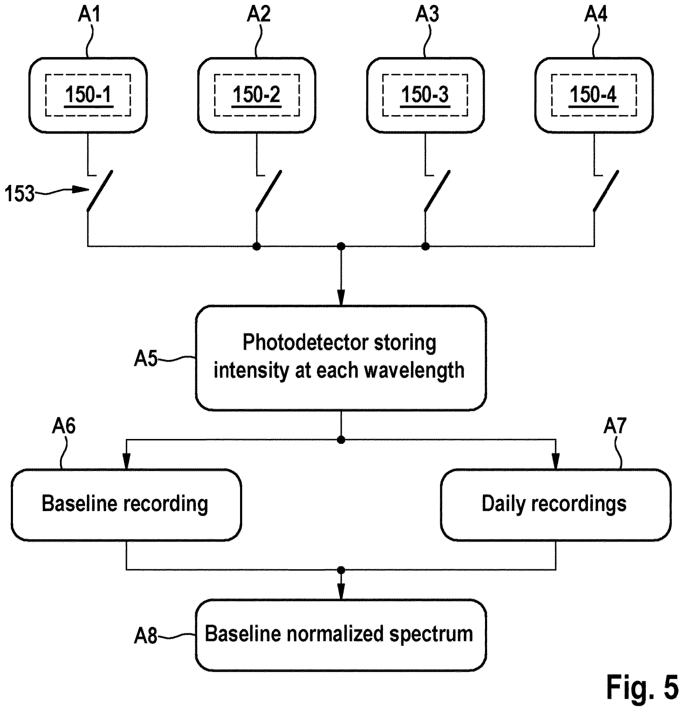

Referring now to Fig. 5, within an infection indication measurement the different light emitting devices 150-1... 150-4, in the example of Fig. 3, are sequentially controlled, one after the other, to each produce a light emission signal at a particular wavelength or in a particular wavelength range e.g. by a switching arrangement 153 (blocks A1 to A4 in the flow diagram of Fig. 5), upon which the light-detecting device 151 senses corresponding light detection signals and outputs output values indicative of the light intensities of received light detection signals (block A5 in Fig. 5). Such infection indication measurements may be conducted e.g. daily (block A7) in order to repeatedly determine an infection indication reading indicative of an infection at the implantation site.

The infection indication may be determined by relating output values of the light-detecting device 151 as obtained during an infection indication measurement to a set of reference values, which are obtained in an initial calibration measurement (block A6). Such a calibration measurement may for example be conducted upon the initial implantation of the implantable medical device 1 in a patient P and shall indicate optical properties in a healthy, non-infected state of the implantation site within the patient P.

Within the calibration measurement, the light emitting devices 150-1... 150-4 are controlled to generate light emission signals, in response to which the light-detecting device 151 senses light detection signals and outputs corresponding output values indicative of the light intensities of the received light detection signals. Such output values are stored as reference values (block A6) and are used in later infection indication measurements for determining an infection indication based on the output values as obtained during a later infection indication measurement (block A7 and block A8).

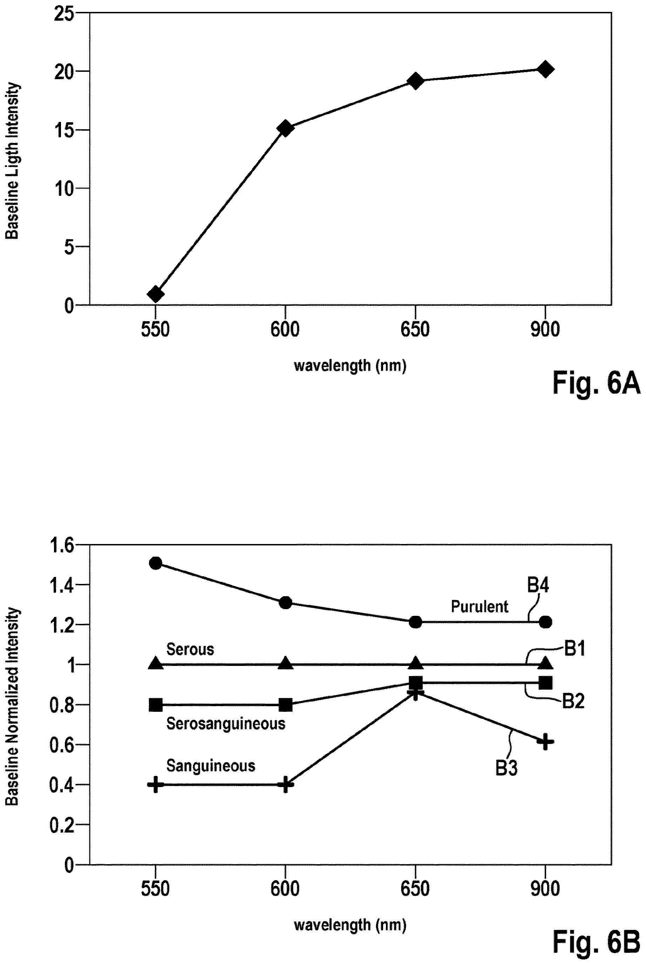

Referring now to Fig. 6A, as a result of a calibration measurement output values indicative of the light intensity at different wavelengths (550 nm, 600 nm, 650 nm, and 900 nm) are obtained, the curve of Fig. 6A representing a baseline curve obtained for example after initial implantation of the implantable medical device 1. The values of Fig. 6A serve as reference values for later infection indication measurements.

Referring now to Fig. 6B, following the initial baseline measurement resulting in the reference values of Fig. 6A, infection indication measurements may repeatedly be conducted, for example once per day, in order to continuously monitor a potential occurrence of an infection at the implantation site of the implantable medical device 1. During an infection indication measurement output values of the light-detecting device 151 indicative of received light intensities at the different wavelengths are obtained and are normalized using the reference values of the baseline curve of Fig. 6A.

Depending on the state at the implantation site, optical transmission and reflection properties at the implantation site may change.

For example, in a normal, non-infected, healthy state a serous exudate may arise at the implantation site, for example within the pocket in which the implantable medical device 1 is received. The serous exudate (curve B1 in Fig. 6B) generally is transparent and hence does not significantly change the transmission and reflection properties at the implantation site. The corresponding curve B1 indicating the output values of the light-detecting device 151 at the different wavelengths normalized by the reference values of Fig. 6A hence exhibits values throughout the entire spectrum at or close to 1.

If at the implantation site, a serosanguineous exudate occurs (curve B2), the exudate may have a pinkish color, due to a small amount of blood in the exudate for example from a damaged capillary. As this causes a light absorption in particular in a lower wavelength region at 550 nm and 600 nm and a reduced absorption at higher wavelengths above 600 nm, the output values indicative of the light intensities at the different wavelengths are smaller than 1, with a more significant decrease in the lower wavelength region.

In the case of a sanguineous exudate (curve B3), the color of the exudate is more reddish due to the existence of fresh blood e.g. from a damaged vessel deep under the tissue. Due to the higher concentration of blood in the exudate, a significant decrease in the output values indicative of the received light intensities at 550 nm, 600 nm and 900 nm is observed, the decrease at 900 nm, in particular, being due to the presence of oxygenated hemoglobin of fresh blood in the exudate, which exhibits a comparatively strong light absorption in the near-infrared range, i.e. at 900 nm, in comparison to the red color range, i.e. at 650 nm. Correspondingly, a decrease at the wavelength of 650 nm is substantially less.

Curves B1 to B3 are not indicative of an infection at the implantation site, but relate to a normal state (curve B 1) or the occurrence of blood at the infection site (curves B2 and B3).

In case of an infection at the implantation site, a purulent exudate fluid will arise at the infection site, the purulent exudate being opaque and greenish or brownish in color. Consequently, light will scatter in the exudate, hence increasing substantially the portion of reflected light which is sensed by the light-detecting device 151. Accordingly, at all wavelengths of 550 nm, 600 nm, 650 nm and 900 nm an increase in the output values indicative of an increase in the received light intensities is observed, the increase being most significant in the lower wavelength region.

Based on infection indication measurements as outlined according to Fig. 6B, an infection indication may be determined. In particular, a set of resulting output values as indicated by the different curves B1 to B4 in Fig. 6B may be assessed, for example by comparing the different output values to a set of thresholds, such that based on the output values an infection indication may be determined, the infection indication being indicative of the presence of an infection at the implantation site of the implantable medical device 1.

For example, for determining an infection indication, the output values as obtained during an infection indication measurement each may be assessed, wherein if for one, some or all output values it is found that the particular output value deviates from an associated reference value by more than a predefined margin and in a predefined direction (increase or decrease), it is concluded for an infection and a corresponding infection indication indicating the presence of an infection is output.

A predefined margin may for example be a percentage value and may be preprogrammed.

By repeated infection indication measurements, a trend of output values indicative of a change in optical characteristics at the implantation site of the implantable medical device 1 may be observed. Based on the trend the infection indication may be identified and output to trigger an infection alert for enabling a timely infection management.

In addition to obtaining output values indicative of received light intensities at different wavelengths, temperature readings may be taken using the temperature sensor 152 of the optical sensing arrangement 15. Temperature measurement values as obtained with the temperature sensor 152 may be put in relation to the results of an infection indication measurement, such that a temperature reading may be taken into account as an additional parameter for identifying an infection indication.

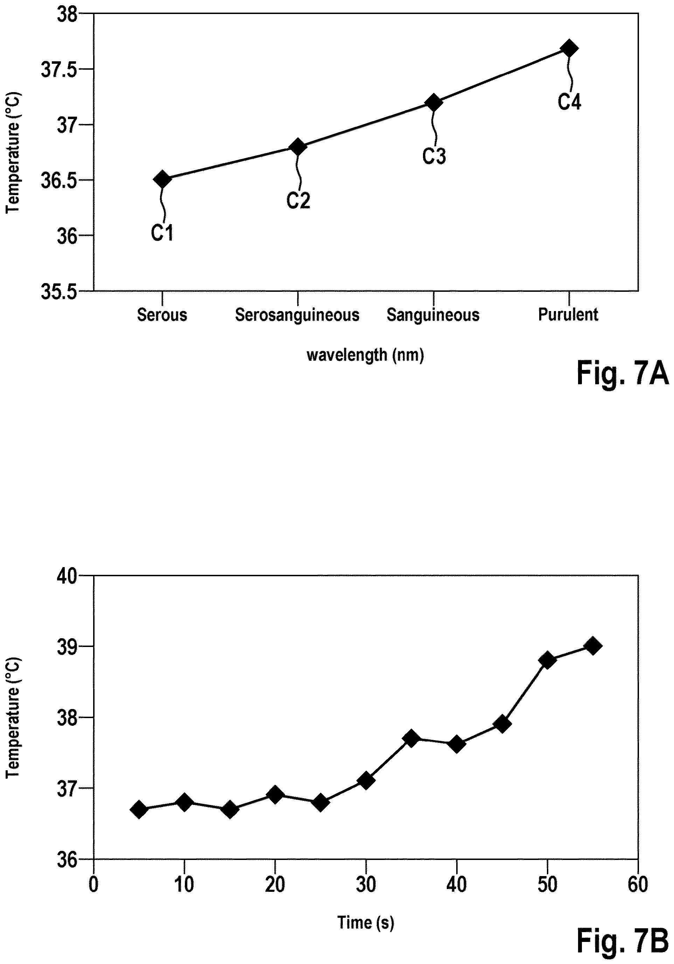

For example, the occurrence of an infection at the implantation site may come with an increase in temperature at the implantation site, as illustrated in Fig. 7A. If for example in an infection indication measurement it is found, based on the optical properties as determined by the output values, that an exudate fluid at the implantation site is a purulent exudate due (curve B4 in Fig. 6) and at the same time a temperature increase is detected (point C4 at the very right in Fig. 7A), the temperature reading supports the finding of infection at the implantation site. If a serous exudate (point C 1 in Fig. 7A), a serosanguineous exudate (point C2 in Fig. 7A), or a sanguineous exudate (point C3) is present, a far reduced temperature rise may be observed.

The use of the temperature sensor 152 may also allow for distinguishing a systemic infection from a local infection at the implantation site. If a temperature increase without a significant change in optical characteristics is observed, this may indicate a systemic infection of the patient P. If, however, the temperature increase comes with a change in optical characteristics, potentially indicating a purulent exudate at the implantation site, this gives rise to the identification of the infection indication indicative of the presence of an infection.

The temperature sensor 152 may also be used to monitor the effects of a potential local heating due to the operation of the optical sensing arrangement 15. If during the time span of an infection indication measurement, which may take place for example in a time span of 60 seconds as illustrated in Fig. 7B, a significant increase in temperature at the temperature sensor 152 is observed, this may indicate a tissue heating in the immediate vicinity of the optical sensing arrangement 15 due to the infection indication measurement, potentially bearing the risk of a thermal harm. If such a temperature increase, as illustrated in Fig. 7B, is detected during the operation of the optical sensing arrangement 15, an infection indication measurement may be terminated and a light emission by light emitting devices 150-1... 150-4 may be stopped.

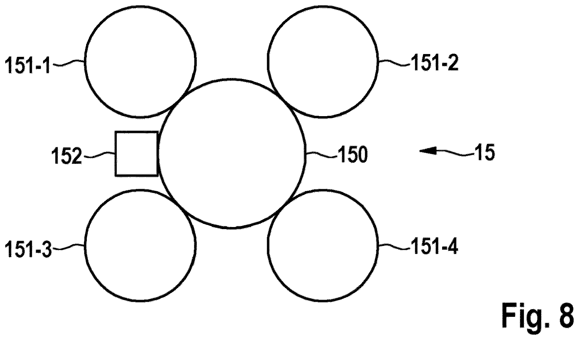

Referring now to Fig. 8, in another embodiment of an optical sensing arrangement 15 a single light-emitting device 150 configured to emit a broadband light signal may be used, in combination with a multiplicity of different light-detecting devices 151-1... 151-4 configured for detecting light detection signals at different wavelengths or in different wavelength ranges. Spectroscopic information in this example hence is obtained by using a multiplicity of different light-detecting devices 151-1... 151-4 associated with different wavelengths or wavelength ranges, wherein signal emission takes place by a single, broadband light emission device 150.

The general processing however remains unchanged. The different light-detecting devices 151-1... 151-4 output values associated with light detection signals at different wavelengths or in different wavelength ranges, such that spectroscopic information indicative of optical transmission and reflection properties at the implantation site at different wavelengths or in different wavelength ranges is obtained.

### List of reference numerals

- 1: Implantable medical device
- 10: Housing
- 11: Electrode arrangement
- 110, 111: Electrode pole
- 12: Processing circuitry
- 13: Energy storage (battery)
- 14: Communication circuitry
- 15: Optical sensing arrangement
- 150, 150-1... 150-4: Light emitting device
- 151, 151-1... 151-4: Light-detecting device
- 152: Temperature sensor
- 153: Switching arrangement
- 2: External device
- A1-A8: Blocks
- B1-B4: Curves
- H: Heart
- I: Infectious region
- L: Light emission signal
- P: Patient
- T: Tissue

## Claims

1. A system for performing a therapeutic and/or diagnostic function in a patient (P), comprising:
an implantable medical device (1) comprising an optical sensing arrangement (15) comprising at least one light-detecting device (151, 151-1... 151-4) for sensing a light detection signal; and
a processing arrangement configured to process an output value of said at least one light-detecting device (151, 151-1... 151-4) indicative of said light detection signal for identifying an infection indication.

2. The system according to claim 1, wherein the implantable medical device (1) comprises processing circuitry (12) implementing at least part of said processing arrangement.

3. The system according to claim 1 or 2, wherein said output value of said at least one light-detecting device (151, 151-1... 151-4) is indicative of a light intensity of said light detection signal.

4. The system according to one of claims 1 to 3, wherein the optical sensing arrangement (15) comprises at least one light-emitting device (150, 150-1... 150-4) for emitting a light emission signal.

5. The system according to one of the preceding claims, wherein the optical sensing arrangement (15) comprises a multiplicity of light emitting devices (150-1... 150-4), wherein a first of the multiplicity of light emitting devices (150-1... 150-4) is configured to emit a first light emission signal at a first wavelength or in a first wavelength range and a second of the multiplicity of light emitting devices (150-1... 150-4) is configured to emit a second light emission signal at a second wavelength or in a second wavelength range different than the first wavelength or the first wavelength range.

6. The system according to claim 5, wherein the multiplicity of light emitting devices (150-1... 150-4) are grouped around a single light-detecting device (151).

7. The system according to one of the preceding claims, wherein the optical sensing arrangement (15) comprises a multiplicity of light-detecting devices (151-1... 151-4), wherein a first of the multiplicity of light-detecting devices (151-1... 151-4) is configured to sense a first light detection signal at a first wavelength or in a first wavelength range and a second of the multiplicity of light-detecting devices (151-1... 151-4) is configured to sense a second light detection signal at a second wavelength or in a second wavelength range different than the first wavelength or the first wavelength range.

8. The system according to claim 7, wherein the multiplicity of light-detecting devices (151-1... 151-4) are grouped around a single light emitting device (150).

9. The system according to one of the preceding claims, wherein the processing arrangement is configured to process output values of said at least one light-detecting device (151, 151-1... 151-4) associated with different wavelengths or different wavelength ranges for identifying said infection indication in an infection indication measurement.

10. The system according to claim 9, wherein the processing arrangement is configured to control said optical sensing arrangement (15) to sequentially obtain, in an infection indication measurement, said output values associated with different wavelengths or different wavelength ranges for identifying said infection indication.

11. The system according to claim 9 or 10, wherein the processing arrangement is configured to associate said output values associated with different wavelengths or different wavelength ranges with a set of reference values for identifying said infection indication.

12. The system according to claim 11, wherein the processing arrangement is configured, in a calibration measurement, to control said optical sensing arrangement (15) for obtaining output values associated with different wavelengths or different wavelength ranges to record said reference values to be used in infection indication measurements subsequent to conducting said calibration measurement.

13. The system according to one of the preceding claims, wherein the optical sensing arrangement (15) comprises a temperature sensor (152) for sensing a temperature measurement value indicative of a temperature in the vicinity of the optical sensing arrangement (15).

14. The system according to one of the preceding claims, comprising an external device (2), wherein the implantable medical device (1) comprises communication circuitry (14) for establishing a communication with the external device (2) for communicating at least one of information relating to output values of the optical sensing arrangement (15) or information relating to processing results for identifying said infection indication to the external device (2).

15. A method for identifying an infection indication in a patient (P), comprising:
sensing, using at least one light-detecting device (151, 151-1... 151-4) of an optical sensing arrangement (15) of an implantable medical device (1), a light detection signal; and
processing an output value of said at least one light-detecting device (151, 151-1... 151-4) indicative of said light detection signal for identifying an infection indication.
